# EUROPEAN PATENT SPECIFICATION

(11) **EP 1 159 441 B1**
(45) Date of publication and mention of the grant of the patent: **17.09.2008**
(21) Application number: 00909483.0
(22) Date of filing: 10.03.2000
(51) Int. Cl.: C12N 15/87, C12N 15/88, C07K 14/035, A61K 47/00

(54) **DELIVERY OF NUCLEIC ACIDS AND PROTEINS TO CELLS**
VERABREICHUNG VON NUKLEINSÄUREN UND PROTEINEN AN ZELLEN
ADMINISTRATION DE SUBSTANCES A DES CELLULES

(30) Priority: 10.03.1999 GB 9905444; 24.12.1999 GB 9930499
(43) Date of publication of application: 05.12.2001
(73) Proprietor: Phogen Limited, Cambridge CB4 0WG (GB)
(72) Inventor: O'HARE, Peter Francis Joseph, Oxted, Surrey RH8 0TL (GB); NORMAND, Nadia Michelle, 92100 Boulogne-Billancourt (FR)
(74) Representative: Kremer, Simon Mark
(86) International application number: PCT/GB2000/000897
(87) International publication number: WO 2000/053722

(56) References cited:
- WO-A-97/05265
- WO-A-97/24453
- WO-A-98/32866
- PHELAN A ET AL: "Intercellular delivery of functional p53 by the herpesvirus protein VP22." NATURE BIOTECHNOLOGY, vol. 16, no. 5, May 1998 (1998-05), pages 440-443, XP002157802 ISSN: 1087-0156

## Description

### Field of the Invention

This invention relates to aggregated compositions for delivery of substances such as nucleic acids and proteins into cells. The invention relates to such compositions in themselves, and to methods for their manufacture and use.

### Background of the invention and prior art

WO 97/05265 (Marie Curie Cancer Care: P O'Hare et al.) relates to transport proteins, in particularVP22 and homologues thereof, and to methods of delivering these proteins and any associated molecules to a target population of cells. This transport protein has applications in gene therapy and methods of targeting agents to cells where targeting at high efficiency is required.

WO 98/32866 (Marie Curie Cancer Care: P O'Hare et al.) discusses coupled polypeptides and fusion polypeptides for intracellular transport, and their preparation and use, e.g. (i) an aminoacid sequence with the transport function of herpesviral VP22 protein (or homologue, e.g. from VZV, BHV or MDV) and (ii) another protein sequence selected from (a) proteins for cell cycle control; (b) suicide proteins; (c) antigenic sequences or antigenic proteins from microbial and viral antigens and tumour antigens; (d) immunomodulating proteins and (e) therapeutic proteins. The coupled proteins can be used for intracellular delivery of protein sequences (ii), to exert the corresponding effector function in the target cell, and the fusion polypeptides can be expressed from corresponding polynucleotides, vectors and host cells.

Elliott and O'Hare (1997) Cell, vol. 88 pp.223-233, relates to intercellular trafficking and protein delivery by a herpesvirus structural protein.

### Summary and description of the invention

The present invention provides aggregated compositions comprising VP22 protein or another polypeptide with the transport function of VP22, and oligonucleotides or polynucleotides.

The aggregated compositions can be formulated as a composition suitable for delivery to cells either ex-vivo, or in culture, or in-vivo as a pharmaceutical composition, for delivery of the polypeptide and/or nucleotide to the cells. Also provided by the invention is a method of intracellular delivery of a polypeptide to a cell which comprises administering to a cell an aggregate as described herein. Also provided is a method of intracellular delivery of a nucleotide to a cell which comprises administering to a cell an aggregate as described herein. The invention further provides a method of expressing a nucleotide in a cell which comprises administering to a cell an aggregate as described herein that comprises a nucleotide that can be expressed and allowing its expression in the treated cell.

According to an aspect of the invention, the mixing of oligonucleotides or polynucleotides with VP22 protein can result in association between the nucleotide and protein to form stable aggregates with particle sizes in the range 0.1-5 microns e.g. 1-3 microns.

Ratios of between 2:1 and 1:1 of protein to nucleotide are most preferred for formation of aggregates. Higher ratios of protein can be used, but lower ratios are less preferred.

By aggregates we mean associations of molecules forming particles of 0.1-5 microns in size e.g. of 1-3 micron in size. 'Aggregate' here is not intended to imply a state of denaturation or inactivity: the aggregates usefully contain active protein and/or functionally active oligo- or polynucleotides.

Oligo- or polynucleotides suitable for forming part of the aggregates of the invention can preferably comprise at least 10 bases(nucleotides) and in length can range widely in size (e.g. in the range 10-50 e.g. 20) e.g. they can be about 4 kilobases in size, and they can comprise plasmids, mini-circles of DNA, or single or double stranded DNA or RNA, or other functionally active nucleotide sequences. Optionally, the nucleotide sequences can also be associated with a DNA condenser, e.g. protamine sulphate.

The VP22 protein referred to can be the native VP22 protein of HSV1 or HSV2. Alternatively, compositions according to the invention can comprise a protein with a sub-sequence less than the whole sequence of the wild-type VP22 protein, that retains the transport functionality of wild-type VP22 protein. Such a sub-sequence can be, for example, a protein corresponding in sequence to amino acid residues 159-301 of VP22. Native VP22 is believed to form stable multimers readily, eitherdimers ortetramers. The sub-sequence based on amino acids 159-301 of VP22 is believed to form dimers readily. The VP22 protein, or protein based on a functional sub-sequence, can further comprise other sequences, e.g. at least one flanking tag fused at the N terminus or at the C terminus of the VP22 or sub-sequence. The tag can be for example, a T7 tag which is an example of an epitope tag enabling antibody detection, e.g. at the N terminus, or it can be for example, a his tag which enables purification of the protein on a nickel containing column, e.g. at the C terminus.

The oligonucleotides or polynucleotides contained in the aggregated composition can be DNA or RNA, that is the nucleotides contained therein can have either an RNA structure wherein the sugar is ribose, or they can have the structure found in DNA wherein the sugar is deoxyribose. When the nucleotides forming the aggregates are RNA, the ribose sugar can be 2'-O-methylated for increased nucleotide stability. In certain examples, the nucleotides can comprise negatively charged modified derivatives of nucleotides e.g. phosphonate derivatives or phosphorothioate derivatives.

In an embodiment of the invention the aggregates can form part of a streptavidin-biotin complex in which the oligo- or polynucleotide is labelled with biotin, e.g. at the 5' end, and this can then be mixed with streptavidin, e.g. streptavidin Alexa 594 (TM), which is streptavidin bound to a fluorophore molecule. Preferably, the streptavidin molecule is modified so that it can be coupled to a molecule, e.g. a drug, which it is desired to deliver to cells , e.g. so that it comprises a disulphide bond which can be used to link it to a molecule which it is desired to deliver to cells and thereby promote subsequent release of the molecule within the cell by intracellular cleavage of the disulphide bond.

Aggregates containing nucleotides such as phosphorothioate derivatives can be of good stability in serum, in spite of the presence of Dnases in serum. They can also be stable in high concentrations of denaturants such as urea, e.g. 7M urea.

Where the oligo- or polynucleotides contain phosphorothioate or other modified nucleotide units as mentioned above, they can be especially stable against degradation by components of serum.

The oligo-or polynucleotides contained in the aggregated compositions can contain ordinary nucleotide phosphodiester linkages. Alternatively, e.g. for achieving longer life and stability against hydrolysis, they can contain phosphorothioate linkages in place of phosphodiester linkages.

It can also be useful to label the the oligo- or polynucleotide, for example with a dtectable label to facilitate detection and monitoring of the aggregate. The label can be at either the 5' or at the 3' end of the synthetic nucleotide. For detection or monitoring of the aggregate any label capable of detection can be used, such as radio-label, or a fluorochrome label.

The nucleotide can be a fluorescent-labelled 20 base oligonucleotide (20-mer) containing phosphorothioate linkages. It can be labelled at the 5' end with 5' fluorescein phosphoroamidite (Genosys), or at the 3' end with fluorescein (Genosys), or at the 5' end with a terminal fluoresceinyl-base (Life Technologies). Also usable is a Texas Red labelled 20mer phosphorothioate that is labelled at the 5' end or 3' end with Texas Red (Genosys).

Aggregates according to the invention can be used to deliver their constituents into target cells.

Cells to which the aggregates can be delivered can be cells of a tissue or an organ in a mammalian subject e.g. a human subject, or they can be explanted cells, or they can be cultured cells e.g.for production of a desired protein. Cultured cells that can be used include but are not limited to: CHO, COS, HeLa and Vero cells, rat aortic smooth muscle cells (RASMC; obtainable from the American tissue culture collection (ATCC)), human aortic smooth muscle cells (HASMC; obtainable from the ATCC), T24 human bladder carcinoma cells (obtainable from the ATCC), RAW 246 macrophage cells, A549 human caucasian lung carcinoma cells (obtainable from the European collection of cell culture), KB-3-1 human cervix carcinoma cells (derived from HeLa cells and obtainable from German collection of cell cultures (DSMZ)), and KB-v1 human cervix carcinoma cells (derived from HeLa cells and obtainable from German collection of cell cultures (DSMZ)) .

In certain examples, when the composition comprises a protein or peptide fused to VP22, or to a sub-sequence thereof, the protein or peptide can be any which can generate an antibody or CTL immune response. Thus the compositions of the invention can be immunogenic compositions, for example they can be vaccines, e.g. DNA or protein vaccines, or both.

In certain examples, the VP22 protein can usefully be a fusion protein in which the protein fusion partner possesses enzymatic activity. For example, a VP22-TK fusion protein, can be used in the compositions e.g. where the target cells are cancer cells e.g. neuroblastoma cells. The compositions can be delivered to target cells, and this can be followed by treatment of the target cells with ganciclovir or equivalent drugs, whereby the TK activity in the composition transported into the cell activates the ganciclovir for cell killing in per se known manner.

It can also be useful to deliver proteins of the compositions for corrective protein therapy.

It can also be useful where VP22, or a sub-sequence thereof, is fused to a cell targeting peptide, such as a peptide that binds to a cell surface receptor, to facilitate cell specific targeting of the complex, e.g. VP22 can be fused to a tumour targeting molecule such as transferrin, or folate. Alternatively, VP22, or a sub-sequence thereof can usefully be fused to a peptide comprising an amino acid sequence which consists of the amino acids arginine, followed by glutamine and aspartate (also known as an RGD motif ; SL Hart, et al., 1996, Gene Therapy 3, pp 1032-1033) and used to target epithelial and endothelial cells. Alternatively, VP22 can be conjugated, using standard methods known in the art for conjugation of sugars to proteins some of which are described in N Sdiqui et al., 1995, Drug delivery 2, pp 63-72 and E Bonifils et al., 1992, Bioconjugate Chemistry 3, pp 277-284, e.g. to a glycoside or lectin molecule such as those mentioned in N Sdiqui et al., 1995, Drug delivery 2, pp 63-72 and E Bonifils et al., 1992, Bioconjugate Chemistry 3, pp 277-284, to facilitate targetting of certain lectin expressing cells, e.g. lectin expressing tumour cells, macrophages, hepatocytes and parenchymal cells.

The oligonucleotide or polynucleotide contained in the aggregated composition according to the invention can be a substance which it is desired to deliver to a target cell.

Forexample, the oligonucleotide or polynucleotide can be single stranded DNA or RNA, such as a 20mer, and it can have a base sequence that enables it, or its transcription product, to function as an antisense or ribozyme molecule in per se known manner, in effect to suppress functional expression of a chosen gene. For example the polynucleotide can be the synthetic hammerhead ribozyme, or any functional homologues or modifications thereof, which can recognise and cleave c-myb RNA, and thereby inhibit cell proliferation (Jarvis et al., J. Biol. Chem., 1996, 271, 29107-29112).

Alternatively, the oligo - or polynucleotide can be antisense in sequence, e.g. antisense to a protein which inhibits apoptosis, such as the Bcl protein, or antiviral antisense e.g. antisense which can bind to a viral AUG start codon or anti-HIV antisense which is complementary to a region of the HIV gag mRNA (J Lisziewicz et al.,1994, PNAS 91, PP 7942-7946), or antitumoral antisense, e.g. antisense to the ras oncogene (G Chen et al., 1996, J Biol Chem 271, pp 28259-28265), or it can be antiparasitic antisense, e.g. trypanasome antisense (P Verspieren et al., 1987, Gene 61, pp307-315). Alternatively, the oligo- or polynucleotide can have the function of correcting splicing defects. The oligo- or polynucleotides can also usefully be chimeroplasts, which are chimeric RNA/DNA oligo- or polynucleotides and which can correct mutations. The oligo- or polynucleotides can also usefully be DNA encoding endogenous ribozymes.

In other examples, the oligonucleotide or polynucleotide can be single stranded DNA of appropriate sequence to enable it to bind to a specific sequence of DNA in the target cell, by forming a triple helix in per se known manner, to block transcription of the gene to which the nucleotide has bound.

In further examples, the oligonucleotide or polynucleotide can be double stranded DNA and can be of appropriate sequence to function as a binding site that binds a specific transcription factor in a target cell, thereby sequestering the transcription factor in the cell (in per se known manner) and suppressing expression of genes that depend for expression on the sequestered transcription factor.

Alternatively or additionally, the protein contained in the aggregated composition according to the invention can be a substance which it is desired to deliver to a target cell. For example, it can comprise VP22 or a protein comprising sub-sequence thereof, or a fusion protein comprising VP22, e.g. for use as a vaccine.

The aggregated compositions according to the invention can also comprise further or other substances for delivery to target cells, such as nucleotides, proteins or peptides fused to VP22.

For example, the aggregated composition can comprise and deliver to a target cell circular or linear DNA of a size sufficient to encode a gene, e.g. to encode a protein. The delivered DNA can also comprise the necessary gene expression elements needed for its expression in the target cell.

In certain examples, the aggregated composition can comprise and deliver single stranded mRNA molecules, of size sufficient to be translated into a protein or peptide, into the cytoplasm of a target cell where the mRNA can be translated into protein or peptide.

In a further aspect of the invention, the VP22 component of the aggregate contains a VP22 sequence and a further component, which can be either the remaining part of a fusion protein, a protein sequence of a desired functionality which it is desired to deliver within the target cell or a nucleotide sequence which it is desired to deliver within the target cell.

The further component can be linked to the VP22 by a cleavage-susceptible amino acid sequence which is susceptible to cleavage by intracellular protease within the target cell. The proteolytic site can be e.g. a site cleaved by a virus encoded protease, such as for example an HIV-encoded protease (D. Serio et al., 1997, PNAS 94, pp 3346-3351) so that cleavage only occurs in virus infected cells, or alternatively the cleavage site can be one which is only cleaved by a cell-specific protease, thereby enabling delivery to a specific cell type. In this aspect of the invention, the fusion protein or coupling product can be delivered within the target cell and cleaved there by protease to release the coupling partner of the VP22, that is, the chosen protein or the nucleotide.

It can also be useful in certain examples to include a coupled protein product that is only active after cleavage of the coupled product in the target cell.

Fusogenic peptides, which can facilitate release from endocytic vesicles within the cell, can also be present in the aggregates according to the invention, e.g. influenza haemagluttinin for selective cell targeting and intracellular delivery. Peptides which can facilitate intracellular targetting can also usefully be present in the aggregates, e.g. the NES peptide (nuclear export signal; L Meunier et al.1999, Nucleic Acids Research 27, pp 2730-2736), e.g.a peptide termed the KDEL peptide (S Seetharam et al., 1991, J Biol Chem 266, pp17376-17381 and U Brinkmann et al., 1991, PNAS 88, PP8616-8620).

It can also be useful to modify the oligo- or polynucleotide so that it can be coupled to a molecule which it is desired to deliver to a cell, for example through a disulphide bridge which can be reduced within the cell and thereby facilitate release of the molecule for delivery.

The aggregates can be delivered to target cells in vivo, such as cells of a tissue or an organ in a mammalian subject, e.g. a human subject. It can for example, be advantageous to deliver aggregates to cancer cells e.g. to introduce an antisense molecule which is of appropriate (per se known) sequence to target a chimeric oncogene, orto suppress a cancergene, e.g. ras or p53, orto suppress an anti-apoptotic gene such as a member of the Bcl gene family.

The aggregates can be delivered to target cells in vivo, by for example, direct injection into target cells, such as a tumour cell mass, or they can be delivered systemically.

Alternatively, the aggregates can be formulated using per se known methods for topical delivery, e.g. for use as part of a therapy for psoriasis, eczema or skin cancer. Alternatively, the aggregates can be encapsulated into slow release capsules suitable for oral delivery using standard methods well known in the art.

The aggregates can also be associated with other delivery systems, for example they can be coupled to liposomes, such as cationic liposomes, or they can be associated with condensing agents, such as DNA condensing agents, e.g. hydrophilic polymers. Among suitable condensing agents are protamine sulphate, and DNA condensing agents such as poly-lysine and histones. They can then be delivered by e.g. direct injection into the target cells, such as tumour cells, or they can be delivered systemically, e.g. using a catheter based approach, or they can be formulated for topical delivery, nasal delivery or oral delivery.

Therapeutic compositions comprising aggregates as described herein can be formulated according to known methods for therapeutically useful compositions, whereby the aggregates are combined in admixture with a pharmaceutically acceptable carrier. Suitable vehicles and their formulation are described in Remingtons Pharmaceutical Science by E.W. Martin (Mack Publishing Company, 1990). The active ingredients are often mixed with pharmaceutically acceptable excipients compatible with the active ingredient. In addition, if desired, the compositions may contain minor amounts of auxiliary substances such as other stabilisers and/or pH buffering agents.

The VP22 component of the aggregates can be stored for long periods at - 70 deg C, for example in a solution of PBS, or alternatively it can be lyophilised and re-constituted before use. The oligonucleotide component of the aggregates can be stored for long periods at - 20 deg C or at 4 deg C, for example in a solution ofTris buffer (pH 7.0 or preferably pH7.5). The VP22 and oligonucleotide components can then be mixed at room temperature for at least 10 mins to enable formation of aggregates according to the invention just prior to delivery of aggregates to cells.

The aggregates can be delivered to target cells which are cells cultured in vitro, for example to CHO, COS, HeLa and Vero cells. The cultured cells containing the aggregates can be used, for example, for target validation in in- vitro testing of gene expression products.

In other embodiments, cells treated with compositions according to the invention can be explanted cells and can then be re-introduced in vivo, e.g. into a mammalian subject.

The aggregates can be substantially resistant to trypsinisation of cultured cells containing them. Therefore cells containing the aggregates in culture can be trypsinised prior to use.

In a further aspect of the invention, exposure to light such as fluorescent light or visible (white) light can be used to promote more rapid disaggregation of the aggregates. For example, after internalisation of the aggregates, target cells in vitro can be exposed to fluorescent light, and where those cells are in vivo they can be exposed to a lasere.g. during photosurgery. When the target cells are cultured cells it can also be useful to produce a cell suspension prior to illumination of the cells, e.g. by trypsinisation of the cells in culture using per se known methods, as cells in suspension can be illuminated for a shorter time period than adherent cells to promote disaggregation of the aggregates.

The aggregated compositions can also comprise a photosensitising molecule, e.g. fluoroscein, rhodamine, or TRITC, which can be linked to the 5' or 3' end of the synthetic nucleotide. This can facilitate the disaggregation of the aggregates in the presence of irradiation, e.g. during phototherapy, for example, as part of a treatment for skin cancer or psoriasis. Irradiation can be achieved in vivo, for example, by introducing into a patient to be treated an endoscope comprising laser optic lines for emitting radiation. Dissociation of aggregates can also be facilitated in the absence of light by introduction of a cleavage site, such as a protease site, or a fusogenic peptide, e.g. the FLU fusion peptide.

Aggregates according to the invention can be useful as cell delivery systems for substances such as proteins or nucleotides, fused with VP22 protein, or a functional part thereof, and can enable delivery into target cells of large amounts of protein or nucleotides.

Following exposure of a cell population to such aggregates, they can be taken up by the cells and the VP22 fusion protein can cause transport to the cell nucleus.

Once the aggregates are taken up into a cell they have been observed in certain examples to remain within the cell for some days, and can also resist cell trypsinisation.

Also provided by the invention is a method of making such aggregates, comprising (a) mixing a VP22 protein or a suitable sub-sequence thereof as mentioned above, optionally fused or covalently coupled to a protein sequence or a nucelotide for delivery to a target cell, with an oligonucleotide or polynucleotide followed by (b) incubating the mix obtained in step (a).

The invention also provides a method for transporting substances into cells, comprising contacting target cells with an aggregated composition according to the invention.

The invention in a further aspect also provides a method of producing/purifying a preparation of the VP22 protein, or a sub-sequence thereof, e.g. a sub-sequence comprising amino acids 159-301 of VP22, comprising treating the protein by affinity chromatography or ion exchange, e.g. using DEAE Sepharose, and (e.g. in a subsequent stage) by purification on a nickel-NTA column.

Examples of the invention are described below without intent to limit its scope.

### Example 1:

This example concerns preparation of an aggregate comprising (i) a fragment of VP22, herein designated 159-301 protein, and consisting of amino acids 159-301 of the VP22 sequence of HSV2 VP22 protein along with (in this example) a his6 tag at the C-terminal end, (ii) and an oligonucleotide which is a 20mer phosphorothioate (of base sequence CCC CCA CCA CTT CCC CTC TC; from Genosys) labelled at the 3' end with fluorescein.

The 159-301 protein can be prepared for example as follows:

159-301 protein can be made in an E.coli expression system expressing a plasmid encoding 159-301 protein, which is a PET-based plasmid containing an IPTG sensitive promoter. The his tag is placed at the C terminus of the protein.

50 ml of bacterial culture expressing the plasmid mentioned above can be grown in nutrient broth suitable for the growth of E. coli, such as L nutrient broth (Oxoid), and also containing kanamycin and chloramphenicol. The recombinant bacteria can be induced by addition of IPTG (0.5mM) to a logarithmic phase culture, and the cells harvested by centrifugation (6000rpm, 4 degC, 20 min). After pelleting the cells can be resuspended in 60 ml of cold lysis buffer containing: 50mM sodium phosphate (pH8.0), 300mM sodium chloride, 5mM imidazole (pH 8.0), 5mM beta-mercaptoethanol, 5 microg/ml Rnase and 5 microg/ml of Dnase-I, 0.5mM PMSF, 1microg/ml of leupeptin, 1microg/ml of pepstatin and 1mg/ml of lysozyme.

The lysis mixture is incubated for 30 min with occasional shaking, and is then sonicated on ice three times for 15 seconds followed by addition of 0.1 % NP-40. Dnase and Rnase are then added to 10 microg/ml and incubated on ice for 20 min with occasional shaking. The lysate is then drawn through a narrow gauge syringe three times. This is followed by centrifugation of the lysate at 14000rpm for 15 min at 4 degC. The supernatant containing the protein is retained.

The 159-301 protein can be purified as follows:

The protein can be partially purified on DEAE sepharose (Pharmacia) followed by centrifugation (3000rpm, 4degC, 5 min) in the presence of lysis buffer comprising 50mM sodium phosphate (pH8), 300mM sodium chloride, 5mM imidazole (pH8), 5mM beta-mercaptoethanol, 5 microgram/ml Rnase and 5 microgram/ml Dnase, 0.5mM PMSF and 10% glycerol, 0.1% NP-40,40mM imidazole (pH8.0), and 1 microgram/ml leupeptin and 1 microgram/ml pepstatin.

The supernatant obtained can then be further purified on a nickel-NTA column. Unbound protein can be discarded, and the column is then washed in wash bufferwhich has the same composition as lysis buffer except that it contains 10% glycerol, 0.1 % NP-40,40mM imidazole (pH8.0), and lacks RNase and DNase. Bound protein is then eluted in eluate buffer which has the same composition as lysis buffer except that it contains 10% glycerol, 0.1 % NP-40, 500mM imidazole (pH8.0), and lacks RNase and Dnase. Alternatively, the protein can be eluted in buffer comprising increasing concentrations of imidazole, e.g. concentrations of imidazole from about 40mM to about 500mM.

The 159-301 protein in solution in eluate buffer is used for the formation of the aggregates. Alternatively, it can be dialysed for 12 hours in PBS before use.

Aggregates can be produced as follows:

25 microlitres of 20mer phosphorothioate-linked oligonucleotide as described above (10micromolar solution in PBS) labelled at the 5' end with fluorescein is added to 25 microlitres of 159-301 protein solution in PBS (20 micromolar solution which contains approximately 150mM sodium chloride and 10mM phosphate at a pH between 7 and 7.2). The final concentration of 159-301 protein in 50 microlitres of PBS is about 10 micromolar and the final concentration of oligonucleotide is about 5 micromolar. The mixture is mixed and left at least 10 min at room temperature. Fifty microlitres of this mixture is then added to 450 microlitres of tissue culture medium (with or without added)serum and can be stored at about 4degC.

The formation of the aggregates of the invention can be monitored by using microscopy e.g. phase contrast or fluorescence microscopy, or by agarose gel electrophoresis of the aggregates.

Aggregates can be delivered to cells as follows:

Aggregates produced by the method previously described can be diluted in pre-warmed tissue culture medium and then added to HeLa cells and incubated for about 12 hours at a temperature of 37degC.

### Example 2:

An aggregate can be made by a method similar to that described in Example 1, except that the oligonucleotide used in the preparation is a oligonucleotide which is a 40mer phosphorothioate labelled at the 5' end with Texas red and with a base sequence as follows:
5' TCC TCG CCC TTG CTC ACC ATG GTG GCG ACC GGT GGA TCC C 3'

This sequence is commercially available and is complementary to a segment of GFP mRNA.
Monitoring of the formation of the aggregates and delivery of the aggregates to cells can be carried out as described in example 1.

### Example 3:

This example is similar to Example 2, except that the oligonucleotide sequence is as follows:
5' CCC TTG CTC ACC ATG GTG GC 3'.

### Example 4:

This example is similar to Example 1, except that the oligonucleotide sequence is as follows:
5' ACC ATG GTG GCG ACC GGT GGA TCC C 3'.

### Example 5:

This example is similar to Example 1, except in that a) the oligonucleotide sequence is as follows:
5' CCC TTG CTC ACC ATG GTG GC 3',
and b) that the aggregates are added to the cells and are incubated with the cells for about 2 hours at a temperature of 37degC.

### Example 5a:

This example is similar to Example 5, except in that the oligonucleotide is a phosphodiester linked oligonucleotide instead of phosphorothioate and is added to cells in PBS and not cell culture medium.

### Example 6:

An aggregate can be made by a method analogous to that described in Example 1, except that (i) the fragment of VP22 consists of amino acids 159-257 of the VP22 sequence of HSV2 VP22 protein, and (ii) the oligonucleotide is a 20mer phosphorothioate labelled at the 5' end with fluorescein and with a base sequence as follows:
5' CCC CCA CCA CTT CCC CTC TC 3'.
This sequence is commercially available and is complementary to a segment of mRNA encoding an intracellular- adhesion molecule, or ICAM.
The 159-257 protein can be prepared and purified as described in Example 1 for preparation and purification of the 159-301 protein, except for the use of an otherwise corresponding plasmid encoding 159-257 protein.
In the aggregates produced, final concentrations of protein and oligonucleotide in 50 microlitres of solution can be about 13.5 micromolar protein and 5 micromolar oligonucleotide.

### Example 7:

An aggregate can be made by a method analogous to that described in Example 1, except that (i) The VP22 '159-301' protein is present as a fusion with the BH3 domain of the bak protein, and (ii) the oligonucleotide is labelled at the 5' end with FITC. A BH3-VP22 '159-301' protein fusion protein can be made as follows:

A double stranded oligonucleotide with the following sequence corresponding to BH3 can be made and cloned into the Bam H1 site of the VP22 '159-301' expression plasmid used to encode the VP22 '159-301' protein, as mentioned above in Example 1:

5'GATCCTATGGGGCAGGTGGACGGCAGCTCGCCATCATCGGGGACGA
CATCAACCGACGCTATCGG

5'GATCCCGATAGCGTCGGTTGATGTCGTCCCCGATGATGGCGAGCTGCC
GTCCCACCTGCCCCATG

The above strands are complementary such that the sequence of the first strand from the seventh residue (adenine) in the 5' to 3' direction is complementary with the sequence of the second strand from the second residue from the end (thymine) in the 3' to 5' direction.

BL21 E. coli cells can be transformed with this BH3-VP22'159-301' expression plasmid, and are grown, induced and the cells harvested as described in Example 1.

After harvesting the cells can be resuspended in 40ml of cold lysis buffer containing: 50mM sodium phosphate (pH 8.0), 300mM sodium chloride, 5mM imidazole (pH 8.0), 5mM beta-mercaptoethanol, 1 microg/ml of leupeptin, 1microg/ml pepstatin and 1 mg/ml lysozyme.

The lysis mixture can be incubated for 30 min with occasional shaking, and is then sonicated on ice three times for 15 seconds followed by addition of 0.1 % NP-40. Dnase and Rnase can then be added to 10 microg/ml and incubated on ice for 20 min with occassional shaking. The lysate can then be drawn through a narrow gauge syringe three times. This can be followed by centrifugation of the lysate at 20,000rpm for 15 min at 4degC. The supernatant containing the VP22-BH3 fusion protein can be retained. The BH3-VP22'159-301' fusion protein can be purified as follows:

The protein can be enriched on DEAE sepharose (Pharmacia) by using a batch method, in the presence of lysis buffer comprising 50mM sodium phosphate (pH 8.0), 300mM sodium chloride, 5mM imidazole (pH 8.0), 5mM beta-mercaptoethanol, 0.1% NP-40, and 1 microgram/ml leupeptin and 1 microgram/ml pepstatin.

The supernatant can then be further purified on nickel-NTA beads in a batch method. Protein can be bound to the beads at 4degC for 1 h. The beads can then be washed three times for 30 mins in wash buffer of the same composition as lysis buffer except that it contains 10% glycerol, 0.1 % NP-40, 40mM imidazole (pH 8.0). Bound protein can then be eluted three times in 1 ml of eluate buffer each time. The eluate buffer can have the same composition as lysis buffer exceptthat it contains 10% glycerol, 0.1 % NP-40, 500mM imidazole (pH 8.0). The eluate buffer can then be exchanged by PD-10 sephadex column chromatography into PBS, 10% glycerol, 5mM B-mercaptoethanol.

The BH3-VP22'159-301' fusion protein obtained by the method described above can be used in the formation of aggregated compositions using a method analogous to that described in example 1:
22.5 microlitres of BH3-VP22 '159-301' protein in PBS can be added to 2.5 microlitres of PBS and 0.5 microlitres of the oligonucleotide
The final concentration of BH3-VP22 '159-301' fusion protein can be about 18 micrograms per ml and the final concentration of oligonucleotide is about 500nM. Monitoring of the formation of the aggregates and delivery of the aggregates to cells can be carried out as described in Example 1.

### Example 8:

A p27-VP22 '159-301' fusion protein can be made by a method analogous to that described in Example 7 for making a BH3-VP22 '159-301' fusion protein, except for the use of an oligonucleotide with a sequence corresponding to the p27 sequence (GenBank Accession Number U 10906) which can be made and cloned into the Nde I and Bam H1 sites of the VP22 '159-301' expression plasmid.

The p27-VP22 '159-301' fusion protein obtained by the method described above can be used in the formation of aggregates using a method analogous to that described in Example 1:
37 microlitres of p27-VP22 '159-301' protein in PBS can be added to 463 microlitres of PBS and 5 microlitres of the oligonucleotide:

The final concentration of p27-VP22 '159-301' fusion protein can be about 185 micrograms per millilitre and the final concentration of oligonucleotide about 2.5 micromolar.
Monitoring of the formation of the aggregates and delivery of the aggregates to cells can be carried out as described in Example 1.

### Example 9:

An aggregate can be made by a method analogous to that described in example 1, except that the oligonucleotide is a 36mer ribozyme which is a 36mer ribozyme as described by Jarvis et al., J. Biol. Chem. 1996, 271, 29107-29112, which can recognise and cleave c-myb and so inhibit cell proliferation, and which is fluorescein labelled at the 5' end and has the following sequence and can be obtained from Cruachem, Glasgow, UK:
5' GUUUUCCCUGAU GAGGCCGAAAGGCCGAAAUUCUCC 3'.

In this sequence all nucleotides are 2'-0-methyl nucleotides with the exception of the following: U at position U5 which is 2'-0-allyl uridine (i.e. the fifth U residue counting from the 5' end of the sequence), G at positions G2, G3 and G9, A at positions A1 and A8 are 2'hydroxyl (ribo)nucleotides. The U at position U5 indicates 2'-0-allyl uridine, whereas the ribozyme described by Jarvis et al. had a 2'-C-allyl uridine linkage at this position ( this being the only difference between the ribozyme described here and that of Jarvis et al.). 5 phosphorothioate linkages are present at the 5' and 3' ends, other linkages are phosphodiester.
Aggregates can be produced by adding the 36mer oligonucleotide to the VP22 '159-301' protein solution in PBS as previously described in Example 1, so that the final concentrations in 50 microlitres of solution can be about 18 micrograms per ml (or alternatively about 32 micrograms per ml) protein and about 500nM oligonucleotide.
Monitoring of the formation of the aggregates and delivery of the aggregates to cells can be carried out as described in Example 1.

### Example 10:

An aggregate can be made as described in example 9, except that the oligonucleotide sequences differs as follows: the second G residue (counting from the 5' end) has been changed to 2'-0-methyl uridine, and the seventh A residue (counting from the 5' end) has been changed to 2'-0-methyl uridine.

### Example 11:

An aggregate can be made by a method similar to that described in Example 1, except that the oligonucleotide is labelled with biotin at the 5' end and has the following sequence:
5' CCC CCA CCA CTT CCC CTC TC 3', and can be obtained from Sigma Genosys), and the aggregate further comprises streptavidin-Alexa 594, which is a protein-fluorophore, and can be obtained from Molecular Probes, Netherlands.

The aggregates can be prepared as follows: 12.5 microlitres of the biotin labelled oligonucleotide (20 microM in PBS) can be mixed with 12.5 microlitres of streptavidin-Alexa 594 (400 nanoM in PBS) and the mixture incubated for 2 hours at room temperature with occasional stirring. Twenty five microlitres of VP22 protein (360 micrograms per ml in PBS) can then be added to the mixture and this mixture incubated for 10 mins at room temperature.

Alternatively, the aggregates can be prepared as follows: 12.5 microlitres of the biotin labelled oligonucleotide (20 microM in PBS) can be mixed with 12.5 microlitres of VP22 (720 micrograms per ml in PBS) and the mixture incubated for 10 mins at room temperature. Twenty five microlitres of streptavidin-Alexa 594 (200 nanoM in PBS) can then be added to the mixture and this mixture incubated for 2 hours at room temperature.
Formation of the aggregates can be monitored as described in example 1.

Aggregates can be delivered to COS cells using the following method: aggregates can be diluted 10 times in cell culture medium containing 10% serum at final concentrations of about 500nM biotin labelled oligonucleotide, about 10nM streptavidin-Alexa594 and about 18micrograms per ml VP22. The cells can then be incubated with the complexes overnight.

### Example 12:

An aggregate can be made by a method similar to that described in Example 1, except that the nucleotide used in the preparation is an oligonucleotide encoding an antisense sequence complementary to a sequence of the ras oncogene (G Chen et al., 1996, J Biol Chem 271, pp28259-28265) labelled with fluorescein at the 5' end and with the following sequence:
5' CCA CAC CGA CGG CGC CC 3'

Formation of the aggregates can be monitored as described in Example 1. The aggregates can then be delivered to cultured T24 cells human bladder carcinoma cells as described in example 1 for delivery to HeLa cells.

T24 cells incubated with the aggregates as described above can then be illuminated for 10 minutes with visible (white light) using a fibre optic cold light (Schott KL 2500 LCD obtainable from Schott Fibre Optics Ltd., Doncaster, UK).

The extent of proliferation of the illuminated T24 cells can then be determined using the crystal violet assay described in N Sdiqui et al., 1995, Drug delivery 2, pp63-72.

Treatment of T24 cells by incubating with aggregates comprising the ras antisense sequence, followed by illumination of the cells as described above, can reduce cell proliferation.

### Example 13:

An aggregate can be made and delivered to T24 cells as described in example 12.

A suspension of T24 cells can then be made by treating the cultured cells with trypsin using per se known methods for trypsinisation of cultured cells , followed by washing of the trypsinised cells. The cell suspension so produced can then be illuminated for 3 minutes with white light.

Reduction of cell proliferation can then be determined as follows: the illuminated cell suspension can then be plated onto cell culture plates. The plated cells can then be trypsinised and the number of cells counted under a microscope.

Treatment of T24 cells by incubating with aggregates comprising ras antisense DNA, followed by trypsinising the cells to obtain a cell suspension and then illumination of the suspension as described above, can reduce T24 cell proliferation.

### Example 14:

An aggregate can be made by a method similar to that described in Example 1, except that the nucleotide used in the preparation is an oligonucleotide encoding an antisense sequence complementary to a sequence of the gene encoding human C-raf kinase (B Monia et al., 1996, PNAS 93, PP 15481-15484), which is a cancer associated gene, labelled with fluorescein at the 5' end and with the following sequence:
5' TCC CGC CTG TGA CAT GCA TT 3'

The aggregates can then be delivered to HeLa cells as described in example 1. A HeLa cell suspension can then be made and illuminated as described in example 13, for T24 cells. Reduction of cell proliferation can be determined as described in example 13.

Treatment of HeLa cells by incubating with aggregates comprising raf antisense DNA, followed by trypsinising the cells to obtain a cell suspension and then illumination of the suspension as described above, can reduce HeLa cell proliferation.

### Example 15:

Aggregates can be made as described in example 14, and delivered to A549 cells as described in example 1, for delivery to HeLa cells.

An A549 cell suspension can then be made and illuminated as described in example 13, for T24 cells. Reduction of cell proliferation can be determined as described in example 13.

Treatment of A549 cells by incubating with aggregates comprising raf antisense DNA, followed by trypsinising the cells to obtain a cell suspension and then illumination of the suspension as described above, can reduce A549 cell proliferation.

## Claims

1. An aggregated composition comprising particles of 0.1 to 5 microns in size, comprising (a) a herpesviral VP22 polypeptide, or subsequence thereof, having the transport function of VP22, and (b) an oligonucleotide or polynucleotide, wherein the aggregated composition is not a herpesvirus particle.

2. An aggregated composition according to claim 1, which further comprises a pharmaceutically acceptable excipient.

3. An aggregated composition according to claim 1 or 2, wherein the polypeptide is a VP22 fragment comprising amino acid residues 159-301 of VP22.

4. An aggregated composition according to any one of the preceding claims, wherein the oligonucleotide or polynucleotide comprises a circular plasmid.

5. An aggregated composition according to any one of the preceding claims, wherein the oligonucleotide or polynucleotide contains modified phosphodiester linkages.

6. An aggregated composition according to claim 5, wherein the modified phosphodiester linkages comprise phosphorothioate linkages.

7. An aggregated composition according to any one of the preceding claims, wherein the oligonucleotide or polynucleotide is labelled with a detectable label.

8. An aggregated composition according to any one of the preceding claims, wherein the polypeptide having the transport function of VP22 is a fusion protein which also comprises a non-VP22 polypeptide sequence, e.g. an immunogenic protein or peptide, a peptide or protein with enzymatic activity, or a cell targeting peptide.

9. An aggregated composition according to claim 8, wherein the non-VP22 polypeptide sequence is linked to the polypeptide having the transport function of VP22 by a cleavage-susceptible amino acid sequence.

10. An aggregated composition according to any one of the preceding claims, wherein the polypeptide or fusion polypeptide is conjugated to a glycoside.

11. An aggregated composition according to any one of the preceding claims, wherein the oligonucleotide or polynucleotide encodes a protein or peptide.

12. An aggregated composition according to any one of claims 1-10, wherein the oligonucleotide or polynucleotide is selected from: an antisense molecule, a ribozyme molecule, a chimeroplast, and a polynucleotide capable of binding a transcription factor.

13. An aggregated composition according to any one of the preceding claims, wherein the oligonucleotide or polynucleotide is coupled to a non-nucleotide molecule, e.g. to a photosensitising molecule.

14. An aggregated composition according to any one of the preceding claims, wherein the aggregate comprises polypeptide and nucleotide in ratios of at least about 1 to 1, e.g. a ratio of about 2 to 1, or polypeptide to nucleotide.

15. An aggregated composition according to any one of the preceding claims, wherein the oligonucleotide or polynucleotide comprises at least about 10 bases, e.g. about 10 bases to about 4 kilobases, e.g. about 10 to about 50 bases, e.g. about 20 bases.

16. An aggregated composition according to any one of the preceding claims, which comprises particles of said aggregate having particle size in the range of 1 to 3 microns.

17. An aggregated composition according to any one of the preceding claims, wherein said polypeptide and said nucleotide are encapsulated in a liposome.

18. A method of making an aggregated composition according to any one of the preceding claims comprising, (a) mixing a herpesviral VP22 polypeptide with the transport function of VP22, with the oligonucleotide or polynucleotide, and, (b) allowing the mixture obtained in step (a) to form aggregates, e.g. aggregates with a particular size of 0.1 to 5 microns, e.g. 1 to 3 microns.

19. A method according to claim 18, wherein the polypeptide is mixed with nucleotide in ratios of at least about 1 to 1, e.g. a ratio of about 2 to 1, of polypeptide to nucleotide.

20. Use of an aggregated composition according to any one of claims 1 to 17, in the manufacture of a medicament suitable for administration to a subject for the purpose of therapy or prophylaxis of disease.

21. Use of an aggregated composition according to claim 13, in the manufacture of a medicament suitable for phototherapy.

22. Use according to claim 20 or 21, wherein the medicament is sterile and is formulated with a pharmaceutically acceptable excipient for parenteral delivery.

23. Use according to claim 20 or 21, wherein the medicament is formulated with a pharmaceutically acceptable excipient for topical, oral or nasal delivery.

24. Use according to any one of claims 20-23, wherein the aggregates are associated with an alkaline DNA binding polypeptide, e.g. protamine.

25. A method of delivering molecules to cells in vitro comprising (a) contacting said cells with an aggregated composition according to any one of claims 1 to 17, optionally followed by, (b) exposing said cells to light to promote disaggregation of said aggregated composition.

## Patentansprüche

1. Aggregierte Zusammensetzung, die Teilchen mit einer Größe von 0,1 bis 5 µm umfasst, umfassend (a) ein Herpesvirus-VP22-Polypeptid oder eine Subsequenz davon, welche(s) die Transportfunktion von VP22 aufweist, und (b) ein Oligonucleotid oder Polynucleotid, worin die aggregierte Zusammensetzung kein Herpesvirus-Partikel ist.

2. Aggregierte Zusammensetzung nach Anspruch 1, die weiters einen pharmazeutisch annehmbaren Exzipienten umfasst.

3. Aggregierte Zusammensetzung nach Anspruch 1 oder 2, worin das Polypeptid ein VP22-Fragment ist, das die Aminosäurereste 159-301 von VP22 umfasst.

4. Aggregierte Zusammensetzung nach einem der vorangegangenen Ansprüche, worin das Oligonucleotid oder Polynucleotid ein ringförmiges Plasmid umfasst.

5. Aggregierte Zusammensetzung nach einem der vorangegangenen Ansprüche, worin das Oligonucleotid oder Polynucleotid modifizierte Phosphodiesterbindungen umfasst.

6. Aggregierte Zusammensetzung nach Anspruch 5, worin die modifizierten Phosphodiesterbindungen Thiophosphatbindungen umfassen.

7. Aggregierte Zusammensetzung nach einem der vorangegangenen Ansprüche, worin das Oligonucleotid oder Polynucleotid mit einer detektierbaren Markierung markiert ist.

8. Aggregierte Zusammensetzung nach einem der vorangegangenen Ansprüche, worin das Polypeptid mit der Transportfunktion von VP22 ein Fusionsprotein ist, das auch eine Nicht-VP22-Polypeptidsequenz, z.B. ein immunogenes Protein oder Peptid, ein Peptid oder Protein mit enzymatischer Aktivität oder ein Zell-Targeting-Peptid, umfasst.

9. Aggregierte Zusammensetzung nach Anspruch 8, worin die Nicht-VP22-Polypeptidsequenz durch eine spaltungsanfällige Aminosäuresequenz an das Polypeptid mit der Transportfunktion von VP22 gebunden ist.

10. Aggregierte Zusammensetzung nach einem der vorangegangenen Ansprüche, worin das Polypeptid oder Fusionspolypeptid an ein Glykosid konjugiert ist.

11. Aggregierte Zusammensetzung nach einem der vorangegangenen Ansprüche, worin das Oligonucleotid oder Polynucleotid für ein Protein oder Peptid kodiert.

12. Aggregierte Zusammensetzung nach einem der Ansprüche 1-10, worin das Oligonucleotid oder Polynucleotid ausgewählt ist aus: einem Antisense-Molekül, einem Ribozym-Molekül, einem Chimeroplast und einem Polynucleotid, das zur Bindung eines Transkriptionsfaktors in der Lage ist.

13. Aggregierte Zusammensetzung nach einem der vorangegangenen Ansprüche, worin das Oligonucleotid oder Polynucleotid an ein Nicht-Nucleotid-Molekül, z.B. an ein Photosensibilisierungsmolekül, gebunden ist.

14. Aggregierte Zusammensetzung nach einem der vorangegangenen Ansprüche, worin das Aggregat ein Polypeptid und ein Nucleotid in Verhältnissen von zumindest etwa 1 zu 1, z.B. einem Verhältnis von etwa 2 zu 1, zwischen Polypeptid und Nucleotid umfasst.

15. Aggregierte Zusammensetzung nach einem der vorangegangenen Ansprüche, worin das Oligonucleotid oder Polynucleotid zumindest etwa 10 Basen, z.B. etwa 10 Basen bis etwa 4 Kilobasen, z.B. etwa 10 bis etwa 50 Basen, z.B. etwa 20 Basen, umfasst.

16. Aggregierte Zusammensetzung nach einem der vorangegangenen Ansprüche, die Teilchen des Aggregats mit einer Teilchengröße im Bereich von 1 bis 3 µm umfasst.

17. Aggregierte Zusammensetzung nach einem der vorangegangenen Ansprüche, worin das Polypeptid und das Nucleotid in einem Liposom eingekapselt sind.

18. Verfahren zur Herstellung einer aggregierten Zusammensetzung gemäß einem der vorangegangenen Ansprüche, umfassend (a) das Vermischen eines Herpesvirus-VP22-Polypeptids, das die Transportfunktion von VP22 aufweist, mit dem Oligonucleotid oder Polynucleotid und (b) das Ermöglichen, dass das in Schritt (a) erhaltene Gemisch Aggregate bildet, z.B. Aggregate mit einer partikulären Größe von 0,1 bis 5 µm, z.B. 1 bis 3 µm.

19. Verfahren nach Anspruch 18, worin das Polypeptid mit einem Nucleotid in Verhältnissen von zumindest etwa 1 zu 1, z.B. einem Verhältnis von etwa 2 zu 1, zwischen Polypeptid und Nucleotid vermischt wird.

20. Verwendung einer aggregierten Zusammensetzung gemäß einem der Ansprüche 1 bis 17 zur Herstellung eines Medikaments, das zur Verabreichung an ein Individuum zum Zweck einer Therapie oder Prophylaxe einer Erkrankung geeignet ist.

21. Verwendung einer aggregierten Zusammensetzung nach Anspruch 13 zur Herstellung eines Medikaments, das für eine Lichttherapie geeignet ist.

22. Verwendung nach Anspruch 20 oder 21, worin das Medikament steril ist und mit einem pharmazeutisch annehmbaren Exzipienten zur parenteralen Verabreichung formuliert ist.

23. Verwendung nach Anspruch 20 oder 21, worin das Medikament mit einem pharmazeutisch annehmbaren Träger zur topischen, oralen oder nasalen Verabreichung formuliert ist.

24. Verwendung nach einem der Ansprüche 20-23, worin die Aggregate mit einem alkalischen DNA-Bindungspolypeptid, z.B. einem Protamin, assoziiert sind.

25. Verfahren zur Verabreichung von Molekülen an Zellen in vitro, umfassend (a) das Kontaktieren der Zellen mit einer aggregierten Zusammensetzung nach einem der Ansprüche 1 bis 17, gegebenenfalls gefolgt von (b) dem Aussetzen der Zellen gegenüber Licht, um die Disaggregation der aggregierten Zusammensetzung zu fördern.

## Revendications

1. Composition agrégée comprenant des particules de 0,1 à 5 microns de sous-dimension comprenant (a) un polypeptide VP22 viral de l'herpès ou sa séquence, ayant la fonction de transport de VP22 et (b) un oligonucléotide, ou un polynucléotide où la composition agrégée n'est pas une particule de virus de l'herpès.

2. Composition agrégée selon la revendication 1, qui comprend de plus un excipient pharmaceutiquement acceptable.

3. Composition agrégée selon la revendication 1 ou 2, où le polypeptide est un fragment de VP22 comprenant les résidus acides aminés 159-301 de VP22.

4. Composition agrégée selon l'une quelconque des revendications précédentes, où l'oligonucléotide ou polynucléotide comprend un plasmide circulaire.

5. Composition agrégée selon l'une quelconque des revendications précédentes, où l'oligonucléotide ou polynucléotide contient des enchaînements phosphodiesters modifiés.

6. Composition agrégée selon la revendication 5, où les enchaînements phosphodiesters modifiés comprennent des enchaînements phosphorothioate.

7. Composition agrégée selon l'une quelconque des revendications précédentes, où l'oligonucléotide ou polynucléotide est marqué au moyen d'un marqueur détectable.

8. Composition agrégée selon l'une quelconque des revendications précédentes, où le polypeptide ayant la fonction de transport de VP22 est une protéine de fusion qui comprend également une séquence d'un polypeptide non-VP22, e.g., une protéine immunogène ou un peptide, un peptide ou une protéine avec une activité enzymatique ou bien un peptide ciblant une cellule.

9. Composition agrégée selon la revendication 8, où la séquence du polypeptide non-VP22 est enchaînée au polypeptide ayant la fonction de transport de VP22 par une séquence d'acides aminés sensible à la scission.

10. Composition agrégée selon l'une quelconque des revendications précédentes, où le polypeptide ou le polypeptide de fusion est conjugué à un glycoside.

11. Composition agrégée selon l'une quelconque des revendications précédentes où l'oligonucléotide ou le polynucléotide code pour une protéine ou un peptide.

12. Composition agrégée selon l'une quelconque des revendications 1-10, où l'oligonucléotide ou polynucléotide est sélectionné parmi une molécule antisense, une molécule de ribozyme, un chiméroplaste et un polynucléotide capable de lier un facteur de transcription.

13. Composition agrégée selon l'une quelconque des revendications précédentes, où l'oligonucléotide ou le polynucléotide est couplé à une molécule non nucléotidique, e.g., à une molécule photosensibilisatrice.

14. Composition agrégée selon l'une quelconque des revendications précédentes, où l'agrégat comprend une polypeptide et un nucléotide à des rapports d'au moins environ 1 à 1, e.g., un rapport d'environ 2 à 1, ou polypeptide à nucléotide.

15. Composition agrégée selon l'une quelconque des revendications précédentes, où l'oligonucléotide ou polynucléotide comprend au moins environ 10 bases, e.g., environ 10 bases à environ 4 kilobases, e.g., environ 10 à environ 50 bases, e.g., environ 20 bases.

16. Composition agrégée selon l'une quelconque des revendications précédentes, qui comprend des particules dudit agrégat ayant une dimension de particule comprise entre 1 et 3 microns.

17. Composition agrégée selon l'une quelconque des revendications précédentes où ledit polypeptide et ledit nucléotide sont encapsulés dans un liposome.

18. Méthode de production d'une composition agrégée selon l'une quelconque des revendications précédentes consiste à (a) mélanger d'un polypeptide VP22 du virus de l'herpès avec la fonction de transport de VP22, avec l'oligonucléotide ou le polynucléotide et (b) laisser le mélange obtenu à l'étape (a) former des agrégats, e.g. des agrégats ayant une dimension de particule de 0,1 à 5 microns, e.g., 1 à 3 microns.

19. Méthode selon la revendication 18, où le polypeptide est mélangé au nucléotide à des rapports d'au moins environ 1 à 1, e.g., un rapport d'environ 2 à 1, du polypeptide au nucléotide.

20. Utilisation d'une composition agrégée selon l'une quelconque des revendications 1 à 17, dans la fabrication d'un médicament approprié pour une administration à un sujet dans le but d'une thérapie ou d'une prophylaxie d'une maladie.

21. Utilisation d'une composition agrégée selon la revendication 13 dans la fabrication d'un médicament approprié pour une photothérapie.

22. Utilisation selon la revendication 20 ou 21, où le médicament est stérile et est formulé avec un excipient pharmaceutiquement acceptable pour une délivrance parentérale.

23. Utilisation selon la revendication 20 ou 21, où le médicament est formulé avec un excipient pharmaceutiquement acceptable pour délivrance topique, orale ou nasale.

24. Utilisation selon l'une quelconque des revendications 20-23, où les agrégats sont associés à un peptide liant l'ADN alcalin, e.g., de la protamine.

25. Méthode de délivrance de molécules à des cellules in vitro comprenant (a) la mise en contact desdites cellules avec une composition agrégée selon l'une quelconque des revendications 1 à 17, facultativement suivie par (b) l'exposition desdites cellules à de la lumière pour favoriser la désagrégation de ladite composition agrégée.
